# EUROPÄISCHE PATENTSCHRIFT

(11) **EP 3 153 186 B1**
(45) Veröffentlichungstag und Bekanntmachung des Hinweises auf die Patenterteilung: **21.03.2018**
(21) Anmeldenummer: 16188141.2
(22) Anmeldetag: 09.09.2016
(51) Int. Cl.: A61L 27/54, A61F 2/02, C09D 5/14, C09D 151/08

(54) **VERFAHREN ZUR HERSTELLUNG EINER ANTIBAKTERIELLEN BESCHICHTUNGSZUSAMMENSETZUNG FÜR IMPLANTATE**
METHOD FOR PRODUCING AN ANTIBACTERIAL COATING COMPOSITION FOR IMPLANTS
PROCEDE DE FABRICATION D'UNE COMPOSITION DE REVETEMENT ANTIBACTERIENNE POUR IMPLANTS

(30) Priorität: 02.10.2015 DE 102015219139
(43) Veröffentlichungstag der Anmeldung: 12.04.2017
(73) Patentinhaber: Gebr. Brasseler GmbH & Co. KG, 32657 Lemgo (DE)
(72) Erfinder: MENZEL, Henning, 31275 Lehrte (DE); WASSMANN, Marco, 37124 Mengershausen (DE); STELLJES, Sebastian, 30449 Hannover (DE); STIESCH, Meike, 30177 Hannover (DE); WINKEL, Andreas, 30163 Hannover (DE)
(74) Vertreter: Hoefer & Partner Patentanwälte mbB

(56) Entgegenhaltungen:
- RU-C1- 2 264 337
- US-A- 5 925 552

## Beschreibung

Die Erfindung betrifft ein Verfahren zur Herstellung einer antibakteriellen Beschichtungszusammensetzung für Implantate mit sehr guter Zellkompatibilität und hervorragender antibakterieller Wirkung. Darüber hinaus betrifft die vorliegende Erfindung auch eine antibakterielle Implantatbeschichtungszusammensetzung sowie ein Verfahren zum Beschichten eines Implantats und ein antibakteriell beschichtetes Implantat.

Implantate werden in vielen medizinischen Disziplinen zur Rekonstruktion von Organ- und Gewebefunktionen eingesetzt. Sie bestehen aus artifiziellen Materialien, an denen Bakterien adhärieren und sich in komplexen Biofilmgemeinschaften organisieren können. Die in der Folge entstehenden Entzündungsreaktionen und damit verbundenen fortschreitenden destruktiven Prozesse im Gewebe führen zu Funktionsverlusten des Implantates und erheblichen Beeinträchtigungen des Patienten. Insbesondere in der Zahnmedizin sind periimplantäre Infektionen mit einer Prävalenz von 30 % von großer klinischer Bedeutung. Aufgrund der hohen Resistenz bakterieller Biofilme gegenüber chemischen Therapeutika erfolgt die Biofilmentfernung in der Zahnmedizin vorwiegend mechanisch, wobei viele Bereiche des zahnärztlichen Implantates aufgrund der komplexen Geometrie nur unvollständig erreichbar sind.

Das Problem der implantatassoziierten Infektionen wird zurzeit durch eine Beschichtung des Implantats mit Silber bekämpft. Die Silberschicht verhindert eine Bakterienadhäsion, allerdings auch die Adhäsion von körpereigenen Zellen, ist also nicht selektiv. Eine Neuentwicklung sind antibakterielle Polymere, die auf die Implantatmaterialien aufgebracht werden. Bisher entwickelte Polymere weisen aber entweder keine ausreichende Zellkompatibilität oder keine hinreichende antibakterielle Wirkung auf. Ein weiteres Problem ist die sichere und technisch umsetzbare Applikation der Polymere auf der Oberfläche.
Die US5925552 beschreibt ein Verfahren zur Herstellung einer Beschichtung auf einem Implantat, wobei (Co-)polymere verwendet werden, die Guanidin-Einheiten besitzen.
Ausgehend von diesem Stand der Technik ist es Aufgabe der Erfindung ein Verfahren zur Herstellung einer antibakteriellen Beschichtungszusammensetzung für Implantate anzugeben, das einfach, ohne hohen technischen Aufwand umsetzbar ist und die Herstellung einer Beschichtungszusammensetzung mit hoher Zellkompatibilität bei sehr guter antibakterieller Funktionalität ermöglicht. Darüber hinaus ist es Aufgabe der vorliegenden Erfindung eine antibakterielle Implantatbeschichtungszusammensetzung bereitzustellen, die einfach applizierbar, auf dem Implantat gut haftend und bei guter antibakterieller Wirkung sehr gut zellkompatibel ist. Eine weitere Aufgabe der vorliegenden Erfindung ist es ein Verfahren zum Beschichten eines Implantats mit einer antibakteriellen Beschichtung anzugeben, das eine gleichmäßig abdeckende, am Implantat anhaftende Beschichtung erzeugt und dabei einen hohen technischen Aufwand vermeidet. Ferner ist es Aufgabe der vorliegenden Erfindung ein antibakteriell beschichtetes Implantat bereitzustellen, das sich durch eine hohe Zellkompatibilität bei sehr guter antibakterieller Funktionalität auszeichnet. Die Lösung dieser Aufgaben erfolgt durch die Merkmale der unabhängigen Ansprüche. Demnach wird die Aufgabe durch ein Verfahren zur Herstellung einer antibakteriellen Beschichtungszusammensetzung für Implantate gelöst, das die folgenden Schritte umfasst: i) umsetzen eines (Meth)Acrylsäure basierten Monomers A, das mindestens ein Epoxid enthält mit einem Polyguanidin, durch Reaktion einer Aminogruppe des Polyguanidins mit dem Epoxid unter Erhalt eines (Meth)Acrylsäure-Polyguanidin-Makromoleküls und ii) polymerisieren des (Meth)Acrylsäure-Polyguanidin-Makromoleküls mit einem mindestens eine polymerisierbare Doppelbindung enthaltenden Monomer B, das mindestens eine Phosphonatgruppe enthält, durch radikalische Polymerisation der (Meth)Acrylsäureeinheit und der Doppelbindung.

Unter allgemein "(Meth)Acrylsäure basierten Verbindungen", wie z.B. "(Meth)Acrylsäure basiertes Monomer A", "(Meth)Acrylsäure-Polyguanidin-Makromolekül", "(Meth)Acrylsäureeinheit", "(Meth)Acrylsäuregruppe" und dergleichen, wird gemäß der Erfindung sowohl eine auf Propensäure, also Acrylsäure, basierte Verbindung als auch eine auf 2-Methylpropensäure, also Methacrylsäure, basierte Verbindung verstanden, nämlich gemäß den o.g. Beispielen: "Methacrylsäure basiertes Monomer A", "Methacrylsäure-Polyguanidin-Makromolekül", "Methacrylsäureeinheit", "Methacrylsäuregruppe". Der Einfachheit halber wird in der nachfolgenden Beschreibung die Abkürzung "(Meth)Acrylsäure ..." in Vertretung für "Acrylsäure ... und "Methacrylsäure ..." verwendet.

Unter Polyguanidinen werden im Sinne der vorliegenden Erfindung Derivate von Oligoguanidinen verstanden, die durch Reaktion von Diaminen mit Guanidinhydrochlorid synthetisierbar sind. Polyguanidine sind zur Bekämpfung von Mikroorganismen bekannt und werden oftmals in Desinfektionsmitteln, Wasch- und Reinigungsmitteln oder Kosmetika eingesetzt. Aufgrund ihrer hohen Wasserlöslichkeit eignen sie sich in der bisher angewendeten Form nicht zur Beschichtung von Implantaten. Durch Reaktion des Polyguanidins mit dem Monomer A, das mindestens ein Epoxid enthält und auf (Meth)Acrylsäure basiert, wird ein polymerisierbares Makromolekül, ein (Meth)Acrylsäure-Polyguanidin-Makromolekül, erhalten, das durch die (Meth)Acrylsäureeinheit eine potentielle Polymerisationsfunktion einträgt. So wird es möglich, mit dem (Meth)Acrylsäure-Polyguanidin-Makromolekül ein weiteres Monomer durch Polymerisation zu verbinden und somit die Wasserlöslichkeit durch Bildung eines polymeren Films herabzusetzen. Das hierfür erfindungsgemäß vorgesehene Monomer B zeichnet sich durch eine hohe Kompatibilität mit dem (Meth)Acrylsäure-Polyguanidin-Makromolekül aus. Durch die Phosphonatgruppe erhält das entstehende Polymer eine Ankerfunktion zum Binden an übliche Implantatmaterialien wie z.B. Titan, Edelstahl, Zirkon, Tantal, Zirkonoxid und dergleichen. Die erfindungsgemäße Beschichtungszusammensetzung ist somit selbstbindend. Es bedarf keiner zusätzlichen adhäsiven Komponente. Dies erleichtert eine Applikation der Beschichtungszusammensetzung auf dem Implantat und ermöglicht Beschichtungen auch auf geometrisch komplexen Implantatoberflächen. Durch die (meth)acrylsäurebasierte Grundstruktur des Monomers A, die Doppelbindungsfunktion des Monomers B sowie das Polyguanidin weist die hergestellte Beschichtungszusammensetzung eine hohe mikrobielle Selektivität auf. Dies bedeutet, dass die Zellkompatibilität bei sehr guter antibakterieller Wirkung hoch ist. Alternative Verfahren, die zunächst ein Anbinden einer Ankerfunktion an eine Implantatoberfläche und eine anschließende Umsetzung mit einer antibakteriell wirkenden Substanz vorsehen, haben hingegen zu keiner ausreichenden Wirkung in Bezug auf eine Keimreduzierung geführt. Die erfindungsgemäß hergestellte Beschichtungszusammensetzung ermöglicht zudem eine gute Adhäsion von humanem Gewebe auf der Implantatoberfläche, was eine Hauptvoraussetzung für die Vermeidung von destruktiven Prozessen ist. Das Verfahren ist dabei ohne hohen technischen Aufwand einfach umsetzbar und erlaubt eine Beschichtung von metallischen und keramischen Implantaten, die mit phosphonathaltigen Gruppen interagieren, auch mit komplexen Geometrien, die einerseits die Besiedlung des Implantats mit pathogenen Keimen signifikant reduziert bzw. vermeidet und andererseits die Adhäsion von körpereigenen Zellen nicht verhindert. So können implantatassoziierte Infektionen effektiv vermeiden werden.

Die Unteransprüche haben vorteilhafte Weiterbildungen und Ausgestaltungen der Erfindung zum Inhalt.

Gemäß einer vorteilhaften Weiterbildung des erfindungsgemäßen Verfahrens ist das Monomer A 2,3-Epoxypropylmethacrylat (Glycidylmethacrylat - GMA). Dieses Monomer hat sich als besonders biokompatibel im Hinblick auf humanes Gewebe herausgestellt und ist sehr gut gemäß dem erfindungsgemäßen Verfahren verarbeitbar. 2,3-Epoxypropylmethacrylat ist sterisch ungehindert und kann somit mit hoher Reaktionsgeschwindigkeit mit dem Polyguanidin reagieren.

Weiter vorteilhaft ist das Monomer B Diethyl(4-vinylbenzyl)phosphonat (VBP), 2-(Dimethoxyphosphoryl)ethylmethacrylat) (DMMEP) oder 2-(Dimethoxyphosphoryl)methylmethacrylat (DMMMP). Diese Monomere erlauben eine sehr schnelle und vollständige Polymerisation mit der Doppelbindung der (Meth)Acrylsäuregruppe im Monomer A. Sie sind gut zellkompatibel und bieten eine stabile Verankerungsfunktion zu üblichen Implantatoberflächen.

Ferner vorteilhaft wird als Polyguanidin Poly-2-(2-ethoxy)-ethoxyethyl-guanidinhydrochlorid (CAS Registry Number 374572-91-5) (PEDBEG) verwendet. Dieses Polyguanidin hat bei guter Zellkompatibilität eine sehr hohe antibakterielle Wirkung.

Eine weitere vorteilhafte Ausgestaltung des Verfahrens sieht vor, dass das PEDBEG durch Umsetzung von Guanidinhydrochlorid mit 1,2-bis(2-aminoethoxy)ethan hergestellt wird und mindestens eine der nachfolgenden Formeln aufweist: 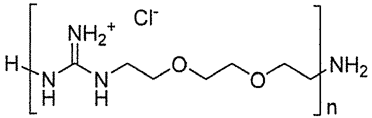 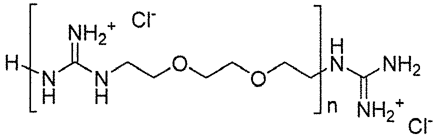 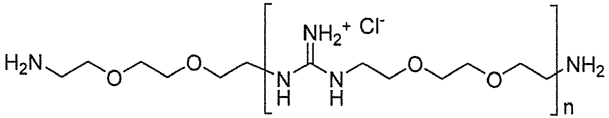 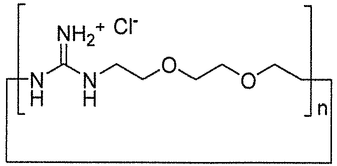 wobei n eine ganze Zahl zwischen 1 und 10, vorzugsweise zwischen 3 und 6, und insbesondere 5 ist. Dieses Polyguanidin zeichnet sich durch eine besonders gute antibakterielle Wirkung bei sehr guter Zellkompatibilität aus.

Die Umsetzung zur Herstellung der Beschichtungszusammensetzung kann dadurch erleichtert werden, dass das Polyguanidin ein Molekulargewicht von 800 bis 1300 g/mol aufweist.

Die Haftung der herzustellenden Beschichtungszusammensetzung an einem Implantat bei im Wesentlichen nicht reduzierter antibakterieller Wirkung kann dadurch verbessert werden, dass ein Anteil des Monomers B bezogen auf die antibakterielle Beschichtungszusammensetzung etwa 67 mol% beträgt.

Ebenfalls erfindungsgemäß wird auch eine antibakterielle Implantatbeschichtungszusammensetzung beschrieben. Die Implantatbeschichtungszusammensetzung wird nach dem vorstehend beschriebenen Verfahren hergestellt, wobei die angeführten vorteilhaften Weiterbildungen des erfindungsgemäßen Verfahrens auch auf die erfindungsgemäße Implantatbeschichtungszusammensetzung Anwendung finden. Wie bereits offenbart, ist es erfindungswesentlich zunächst das Polyguanidin mit dem Monomer A zu einem Makromonomer umzusetzen und erst dann eine Copolymerisation mit dem Monomer B durchzuführen anstatt zunächst Monomere A und B zu copolymerisieren und erst anschließend das Copolymer mit dem Polyguanidin umzusetzen. Obwohl theoretisch für beide Reaktionswege ähnliche Produktstrukturen postulierbar sind, führt nur das erfindungsgemäße Verfahren zu einer Implantatbeschichtungszusammensetzung mit hoher antibakterieller Wirksamkeit. Dies wurde anhand von Beimpfungstests nachgewiesen. Es wird somit davon ausgegangen, dass sich die chemische Struktur der erfindungsgemäßen Implantatbeschichtungszusammensetzung von der Struktur, die mit den gleichen Ausgangssubstanzen aber durch Reaktion in abweichender Reihenfolge erhalten wird, unterscheidet. Die erfindungsgemäß hergestellte Implantatbeschichtungszusammensetzung zeichnet sich bei guter Haftung an herkömmlichen Implantatoberflächen durch eine hohe Zellkompatibilität und gute antimikrobielle Wirksamkeit aus.

Weiter erfindungsgemäß wird auch ein Verfahren zum Beschichten eines Implantats mit einer antibakteriellen Beschichtung beschrieben. Das Verfahren umfasst das Herstellen einer Beschichtungszusammensetzung mit den Schritten i) umsetzen eines (Meth)Acrylsäure basierten Monomers A, das mindestens ein Epoxid enthält mit einem Polyguanidin, durch Reaktion einer Aminogruppe des Polyguanidins mit dem Epoxid unter Erhalt eines (Meth)Acrylsäure-Polyguanidin-Makromoleküls und ii) polymerisieren des (Meth)Acrylsäure-Polyguanidin-Makromoleküls mit einem mindestens eine polymerisierbare Doppelbindung enthaltenden Monomer B, das mindestens eine Phosphonatgruppe enthält, durch radikalische Polymerisation der (Meth)Acrylsäureeinheit und der Doppelbindung unter Herstellung einer Beschichtungszusammensetzung. Die hier dargelegten Verfahrensschritte entsprechen den Verfahrensschritten des erfindungsgemäßen Verfahrens zur Herstellung einer antibakteriellen Beschichtungszusammensetzung. Es wird daher auf die vorstehende Offenbarung des erfindungsgemäßen Verfahrens verwiesen. Die somit erhaltene Beschichtungszusammensetzung wird im Schritt iii) in einem Lösungsmittel gelöst. Das Lösungsmittel ist im Einzelnen nicht beschränkt und richtet sich nach der Löslichkeit der Beschichtungszusammensetzung. Als vorteilhaft haben sich Alkohole wie Methanol, Ethanol und Isopropanol erwiesen. Es wird eine Lösung der Beschichtungszusammensetzung erhalten die auch kolloidal sein kann. In einem weiteren Verfahrensschritt iv) wird ein Implantat bereitgestellt, das entfettet wird. Das Entfetten kann z.B. durch Reinigung mit Lösungsmitteln wie Aceton, Dichlormethan, Methanol und dergleichen oder mittels Plasmareinigung erfolgen. Ggf. nach Trocknung erfolgt anschließend das Aufbringen der Lösung der Beschichtungszusammensetzung auf das Implantat im Schritt v). Dadurch dass die Beschichtungszusammensetzung in Form einer Lösung vorliegt, können viele einfache, herkömmliche Applikationsprozesse Anwendung finden, was den technischen Aufwand für das Verfahren gering hält. Auch wird so eine Applikation auf geometrisch komplexen Oberflächen vereinfacht. Abschließend erfolgt im Schritt vi) das Binden der Beschichtungszusammensetzung an das Implantat. Dies wird durch Einwirken von Temperaturen zwischen 50 und 200 °C z.B. in einem Trockenschrank oder einem geeigneten Ofen ausgeführt. Durch den Bindeschritt zieht die polymere Beschichtungszusammensetzung auf die Oberfläche des Implantats auf und bindet durch die Phosphonatgruppen kovalent an das Implantat. Die Bindungskraft der Bindung zwischen der Implantatoberfläche und der Beschichtungszusammensetzung ist hoch. Das Aufbringen eines Adhäsivs kann somit entfallen. Durch das Verfahren wird ohne hohen technischen Aufwand ein Implantat mit dauerhaft hoher antimikrobieller Wirksamkeit bei sehr guter Zellkompatibilität erhalten.

Die vorstehend für das erfindungsgemäße Verfahren zur Herstellung einer antimikrobiellen Beschichtungszusammensetzung dargelegten Vorteile, vorteilhaften Weiterbildungen und Ausgestaltungen finden auch Anwendung auf das erfindungsgemäße Verfahren zum Beschichten eines Implantats mit einer antibakteriellen Beschichtung.

Zur Aufreinigung der Beschichtung können sich nach dem Binden der Beschichtungszusammensetzung ein Waschschritt und ein Trocknungsschritt anschließen.

Des Weiteren kann das Implantat vor dem Beschichten übliche Vorbereitungsschritte durchlaufen, wie z.B. ein Polieren der Oberfläche, um eine gewünschte Oberflächenrauheit, beispielsweise eine Oberflächenrauheit von 0,013 µm oder darunter, zu erhalten.

Eine vorteilhafte Weiterbildung des Verfahrens sieht vor, dass das Aufbringen der Lösung der Beschichtungszusammensetzung durch Spin Coating (insbesondere bei planaren Substraten), ein Tauch- oder Sprühverfahren ausgeführt wird. Hierdurch wird die Verfahrensführung vereinfacht und eine Beschichtung mit besonders gleichmäßiger Schichtdicke erhalten.

Eine sehr gute Reaktionsfähigkeit der Beschichtungszusammensetzung mit der Implantatoberfläche unter Ausbildung einer ebenmäßigen Schichtdicke wird vorteilhaft dadurch erhalten, dass eine Konzentration der Beschichtungszusammensetzung im Lösungsmittel 2 bis 20 mg/ml und insbesondere 8 bis 12 mg/ml, beträgt.

Des Weiteren erfindungsgemäß wird auch ein antimikrobiell beschichtetes Implantat beschrieben. Das antimikrobiell beschichtete Implantat ist nach dem vorstehend beschriebenen Verfahren zum Beschichten eines Implantats mit einer antibakteriellen Beschichtung hergestellt. Das erfindungsgemäße Implantat ist aus Titan, Zirkon, Tantal, Edelstahl oder Zirkonoxid gebildet und weist eine durchschnittliche Schichtdicke der antimikrobiellen Beschichtung von 5 bis 50 nm und insbesondere von 15 bis 25 nm auf. Die Schichtdicke wird dabei durch Ellipsometrie bestimmt. Die durch das erfindungsgemäße Verfahren im erfindungsgemäßen Implantat erhaltene Schichtdicke liegt um etwa 10 nm höher als diejenige, die durch alternative Herstellverfahren einer Beschichtungszusammensetzung unter sonst gleichen Bedingungen erhalten werden. Dies deutet darauf hin, dass die chemische Struktur der Beschichtungszusammensetzung nicht mit Strukturen der in anderer Sequenz hergestellten Beschichtungszusammensetzungen übereinstimmt. Beispielhafte erfindungsgemäße Implantate umfassen dentale Implantate, Implantate für Hüft- und Knieendoprothesen sowie Herzschrittmacher. Insbesondere in der Tumortherapie, wenn immunsupprimierte Patienten große Implantate erhalten, ist eine Infektionsprophylaxe von enormer Bedeutung. Insbesondere für dentale Implantate, die naturgemäß in Kontakt mit der bakteriell stark belasteten Mundhöhle stehen und deshalb einem besonderen Infektionsrisiko ausgesetzt sind, ist die vorliegende Erfindung besonders geeignet.

Weitere Einzelheiten, Vorteile und Merkmale der vorliegenden Erfindung ergeben sich aus nachfolgender Beschreibung von Ausführungsbeispielen anhand der Zeichnung. Es zeigt:
- Fig. 1: ein Reaktionsschema zur Herstellung einer antimikrobiellen Beschichtungszusammensetzung gemäß einer ersten Ausgestaltung der Erfindung,
- Fig. 2: ein Reaktionsschema zur Herstellung einer antimikrobiellen Beschichtungszusammensetzung gemäß einer zweiten Ausgestaltung der Erfindung,
- Fig. 3: eine Übersicht über mögliche Strukturen von PEDBEG,
- Fig. 4: eine schematische Darstellung von Mikroskopaufnahmen nach Besiedelung mit Staphylococcus aureus, und
- Fig. 5: eine graphische Darstellung der Zellzahl von adhärenten Gingiva-Fibroblasten nach 24 Stunden und 72 Stunden.

Die vorliegende Erfindung wird anhand von Ausführungsbeispielen im Detail erläutert. Dabei werden nur die erfindungswesentlichen Aspekte der Erfindung dargestellt. Alle übrigen Aspekte sind der Übersichtlichkeit halber weggelassen.

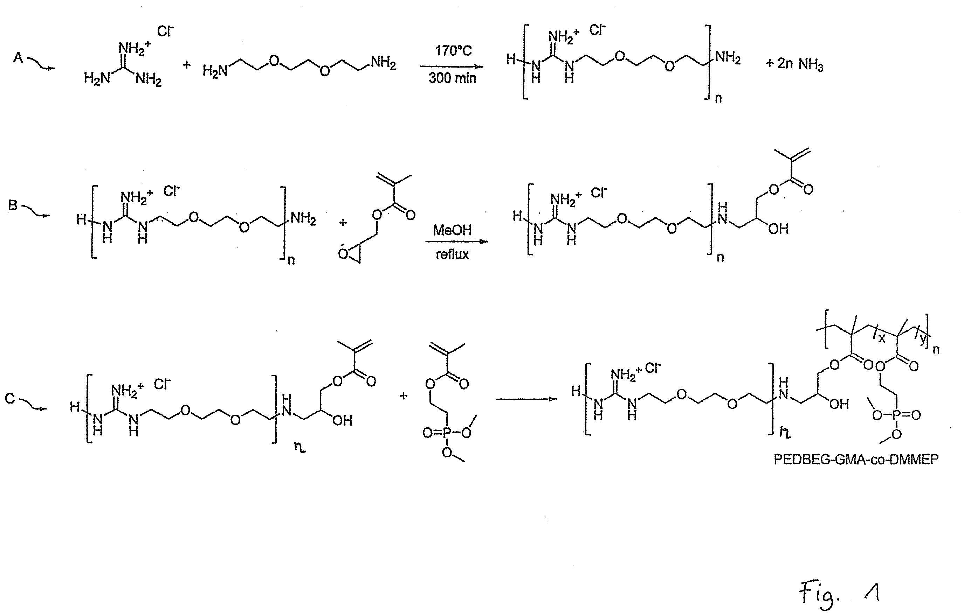

Im Detail zeigt Figur 1 ein Reaktionsschema zur Herstellung einer antimikrobiellen Beschichtungszusammensetzung am Beispiel von Copolymer PEDBEG-GMA-co-DMMEP. Reaktionsschritt A stellt die Synthese von PEDBEG durch Polykondensation von Guanidinhydrochlorid und 2,2-(Ethylendioxy)bis(ethylamin) dar, wobei n vorzugsweise 5 ist. Hierzu werden in einen 50 ml Dreihalskolben mit mechanischem Rührer 50 mmol (7,41 g) 2,2-(Ethylendioxy)bis(ethylamin) und 525 mmol (5 g) Guanidinhydrochlorid gegeben. Die Reaktionsmischung wird innerhalb von 30 min auf 170°C erhitzt und danach für 300 min bei dieser Temperatur unter Stickstoffatmosphäre gerührt. Der während der Reaktion freiwerdende Ammoniak wird durch eine wässrige Salzsäurelösung geleitet und so neutralisiert. Anschließend an die Reaktion wird noch in der Reaktionsmischung befindlicher Ammoniak durch Rühren für 40 min bei 170°C unter Vakuum entfernt. Das Produkt ist eine gelblich-viskose Lösung die beim Erkalten fest wird.

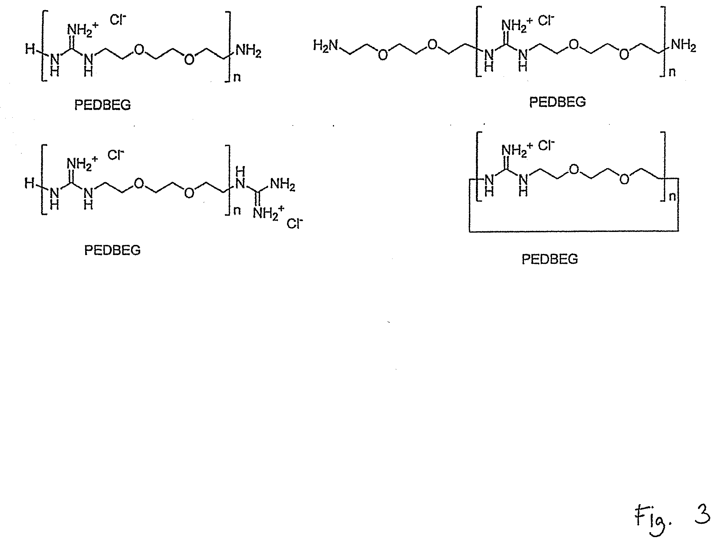

Im Reaktionsschritt B erfolgt die Kupplungsreaktion der im Reaktionsschritt A erhaltenen PEDBEG-Oligomere mit 2,3-Epoxypropylmethacrylat (Glycidylmethacrylat - GMA) (Monomer A) unter Erhalt eines (Meth)Acrylsäure-Polyguanidin-Makromoleküls. Beispielhaft ist nur eine Struktur eines PEDBEG-Oligomers gezeigt. Bezüglich weiterer Strukturen wird ergänzend Bezug genommen auf Fig. 3. In einem 50 ml Zweihalskolben werden 5 mM PEDBEG (5 g) in 20 ml Methanol vorgelegt und dann 150 mg GMA (1,2 mM) zugegeben. Die Lösung wird für 40 Stunden refluxiert. Eine Aufarbeitung erfolgt durch Abziehen des Lösemittels im Vakuum.

Im Reaktionsschritt C wird die Copolymerisation von PEDBEG-GMA mit 2-(Dimethoxyphosphoryl)ethylmethacrylat (DMMEP) (Monomer B) als freie radikalische Copolymerisation in Methanol mit AIBN als Radikalstarter durchgeführt. Dazu wird eine einmolare Lösung des Monomers B in Methanol hergestellt. Außerdem wird eine Initiatorlösung mit 164 mg AIBN auf 10 ml Methanol angesetzt. Die Ansätze haben ein Volumen von 5 ml und werden in einem 1:1 Verhältnis Phosphonat zu PEDBEG-GMA mit je 0,5 ml Initiatorlösung hergestellt. Die Schraubreagenzgläser werden für zwei Minuten mit Stickstoff durchspült um Sauerstoff zu entfernen und danach fest verschlossen. Die Copolymerisation wird bei 60°C für 14 Stunden durchgeführt und durch Eintauchen in Eiswasser abgebrochen. Die Aufarbeitung erfolgt durch Fällen der Polymere in kaltem Diethylether, absaugen über POR2 Fritten und Trocknen im Vakuum für drei Tage bei Raumtemperatur. In dem erhaltenen PEDBEG-GMA-co-DMMEP ist x ungefähr 23 mol% und y ungefähr 67 mol%.

Die erhaltene Beschichtungszusammensetzung, das Copolymer PEDBEG-GMA-co-DMMEP, zeichnet sich durch eine hohe Bereitschaft zum Binden an übliche Implantatoberflächen, wie Titan, Edelstahl, Zirkon, Tantal und Zirkoniumoxid aus.

Zum Anbinden der Beschichtungszusammensetzung an eine Implantatoberfläche wurde PEDBEG-GMA-co-DMMEP als Lösung mit einer Konzentration von 10 g/l in Methanol auf entfettete Proben von Implantatoberflächen (z.B. Titan Grade 5) aufgebracht, zum Binden der Beschichtungszusammensetzung für 16 Stunden bei 120°C gelagert und dann im Ultraschallbad für zweimal 20 min mit dem Lösungsmittel Methanol gewaschen.

Das so hergestellt antimikrobielle Implantat wies sich bei hervorragender antimikrobieller Eigenschaft als sehr gut zellkompatibel aus.

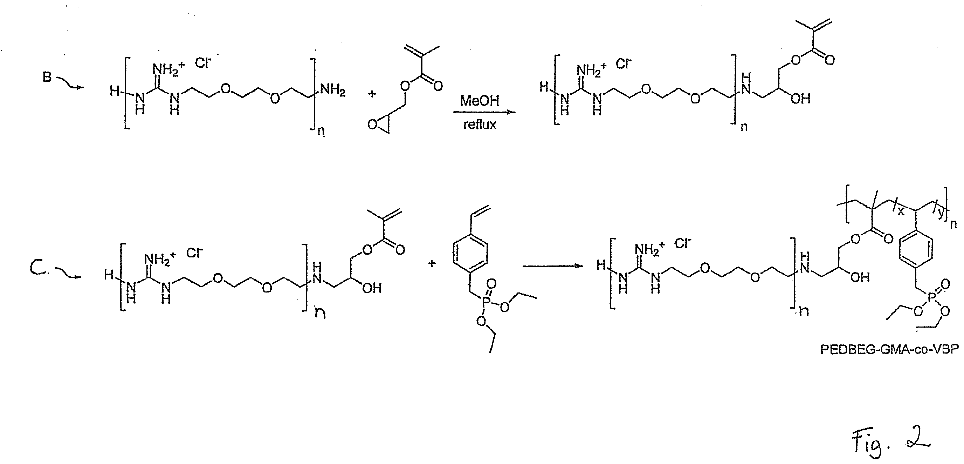

Figur 2 zeigt die Reaktionsschritte B und C aus Figur 1 für eine Copolymerisation von PEDBEG-GMA mit Diethyl(4-vinylbenzyl)phosphonate (VBP). Die chemische Umsetzung erfolgt analog zu den Ausführungen zu Figur 1.

Die gemäß Figur 2 erhaltene Beschichtungszusammensetzung, das Copolymer PEDBEG-GMA-co-VBP, in dem x ungefähr 23 mol% und y ungefähr 67 mol% beträgt, zeichnet sich durch eine hohe Bereitschaft zum Binden an übliche Implantatoberflächen, wie Titan, Edelstahl, Zirkon, Tantal und Zirkoniumoxid aus.

Zum Anbinden der Beschichtungszusammensetzung an eine Implantatoberfläche wurde PEDBEG-GMA-co-VBP als Lösung mit einer Konzentration von 10 g/l in Methanol auf entfettete Proben von Implantatoberflächen (z.B. Titan Grade 5) aufgebracht, zum Binden der Beschichtungszusammensetzung für 16 Stunden bei 120°C gelagert und dann im Ultraschallbad für zweimal 20 min mit dem Lösungsmittel Methanol gewaschen.

Figur 3 ist eine Übersicht über mögliche oligomere Strukturen von PEDBEG, wobei n vorzugsweise 5 ist.

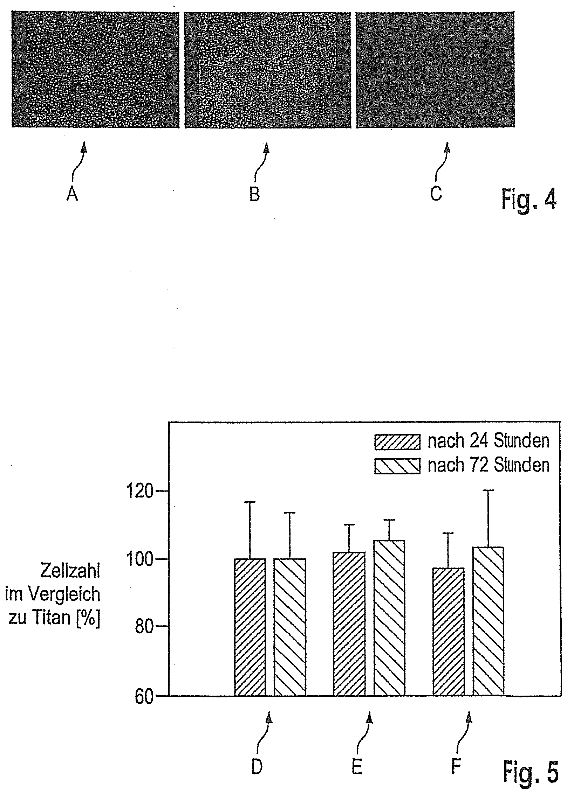

Figur 4 ist eine schematische Darstellung von Mikroskopaufnahmen nach Besiedelung mit Staphylococcus aureus. Dazu wurden Bakterien (Staphylococcus aureus) auf der Oberfläche von Implantatproben A, B und C ausgesät und für 24 Stunden kultiviert. Anschließend wurden die adhärenten Bakterien mit einem Fluoreszenzfarbstoff angefärbt und sichtbar gemacht. Implantatprobe A war Titan (Grade 5) ohne antimikrobielle Beschichtung, Implantatprobe B war mit VBP-GMA+PEDBEG beschichtetes Titan (Grade 5) und Implantatprobe C war mit gemäß dem Reaktionsschema aus Figur 1 hergestellter Beschichtungszusammensetzung (PEGBEG-GMA-co-DMMEP, 67% mol% DMMEP) beschichtet.

Zu Implantatprobe B ist auszuführen, dass zur Herstellung der Beschichtung zunächst VBP mit GMA polymerisiert wurde, das Copolymer auf das Titan angebunden und erst anschließend eine weitere Umsetzung mit PEDBEG erfolgte.

In Figur 4 ist deutlich eine Reduktion der Keimzahl auf der erfindungsgemäßen PEDBEG-GMA-co-DMMEP-Beschichtung erkennbar, während auf der Beschichtung von Implantatprobe B sowie auf Implantatprobe A ein starkes Keimwachstum zu beobachten war.

Weiterhin wurde die Zellkompatibilität von Implantatproben untersucht. Die Ergebnisse sind im Diagramm in Figur 5 dargestellt. Hierzu wurden Implantatproben D, E und F wie folgt hergestellt: Implantatprobe D: Titan (Grade 5), Implantatprobe E (Vergleichsbeispiel): Titan (Grade 5) beschichtet mit GMA-co-VBP+PEDBEG und Implantatprobe F (erfindungsgemäßes Beispiel): Titan (Grade 5) beschichtet mit PEDBEG-GMA-co-DMMEP und mit humanen Gingiva-Fibroblasten (HGFib) besiedelt. Dabei wurden sowohl Adhäsion und Proliferation der Zellen auf den Oberflächen beurteilt. Die Ergebnisse für die Adhäsion wurden nach 24 Stunden und die Ergebnisse der Proliferation nach 72 Stunden untersucht.

Zu Implantatprobe E ist auszuführen, dass zur Herstellung der Beschichtung zunächst VBP mit GMA polymerisiert wurde, das Copolymer auf das Titan angebunden und erst anschließend eine weitere Umsetzung mit PEDBEG erfolgte.

Die Adhäsion von HGFib auf der Implantatprobe E ist auf dem gleichen Niveau wie bei blankem Titan (D). Für die Proliferation ergibt sich ein leicht besserer Wert. Die Adhäsion von HGFib auf der Implantatprobe F ist auf ähnlichem Niveau wie bei blankem Titan (D). Für die Proliferation ergibt sich sogar ein leicht besserer Wert. Die erfindungsgemäße Beschichtungszusammensetzung PEDBEG-GMA-co-DMMEP, beeinflusst somit HGFib-Zellen nicht negativ bei der Adhäsion. Die erfindungsgemäße Beschichtungszusammensetzung zeigte eine exzellente Biokompatibilität.

Neben der vorstehenden schriftlichen Beschreibung der Erfindung wird zu deren ergänzender Offenbarung hiermit explizit auf die zeichnerische Darstellung der Erfindung in den Fig. 1 bis 5 Bezug genommen.

## Patentansprüche

1. Verfahren zur Herstellung einer antibakteriellen Beschichtungszusammensetzung für Implantate, umfassend die Schritte:
• umsetzen eines (Meth)Acrylsäure basierten Monomers A, das mindestens ein Epoxid enthält mit einem Polyguanidin, durch Reaktion einer Aminogruppe des Polyguanidins mit dem Epoxid unter Erhalt eines (Meth)Acrylsäure-Polyguanidin-Makromoleküls und
• polymerisieren des (Meth)Acrylsäure-Polyguanidin-Makromoleküls mit einem mindestens eine polymerisierbare Doppelbindung enthaltenden Monomer B, das mindestens eine Phosphonatgruppe enthält, durch radikalische Polymerisation der (Meth)Acrylsäureeinheit und der Doppelbindung.

2. Verfahren nach Anspruch 1, **dadurch gekennzeichnet, dass** das Monomer A 2,3-Epoxypropylmethacrylat ist.

3. Verfahren nach Anspruch 1 oder 2, **dadurch gekennzeichnet, dass** das Monomer B Diethyl(4-vinylbenzyl)phosphonate, 2-(Dimethoxyphosphoryl)ethylmethacrylat oder 2-(Dimethoxyphosphoryl)methylmethacrylat ist.

4. Verfahren nach einem der vorhergehenden Ansprüche, **dadurch gekennzeichnet, dass** das Polyguanidin Poly-2-(2-ethoxy)-ethoxyethyl-guanidinhydrochlorid ist.

5. Verfahren nach einem der vorhergehenden Ansprüche, **dadurch gekennzeichnet, dass** das Polyguanidin durch Umsetzung von Guanidinhydrochlorid mit 1,2-bis(2-aminoethoxy)ethan hergestellt wird und mindestens eine der nachfolgenden Formeln aufweist: 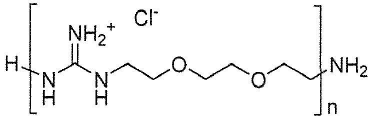 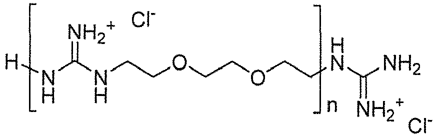 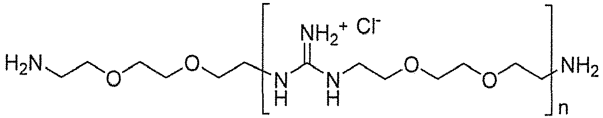 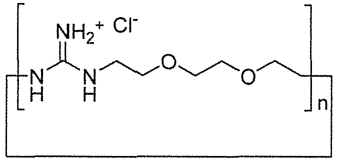 wobei n eine ganze Zahl zwischen 1 und 10, vorzugsweise zwischen 3 und 6, und insbesondere 5 ist.

6. Verfahren nach einem der Ansprüche 4 oder 5, **dadurch gekennzeichnet, dass** das Molekulargewicht des Polyguanidins 800 bis 1300 g/mol beträgt.

7. Verfahren nach einem der vorhergehenden Ansprüche, **dadurch gekennzeichnet, dass** ein Anteil des Monomers B bezogen auf die antibakterielle Beschichtungszusammensetzung etwa 67 mol% beträgt.

8. Antibakterielle Implantatbeschichtungszusammensetzung, hergestellt nach einem Verfahren nach einem der vorhergehenden Ansprüche.

9. Verfahren zum Beschichten eines Implantats mit einer antibakteriellen Beschichtung umfassend die Schritte:
• umsetzen eines (Meth)Acrylsäure basierten Monomers A, das mindestens ein Epoxid enthält mit einem Polyguanidin, durch Reaktion einer Aminogruppe des Polyguanidins mit dem Epoxid unter Erhalt eines (Meth)Acrylsäure-Polyguanidin-Makromoleküls,
• polymerisieren des (Meth)Acrylsäure-Polyguanidin-Makromoleküls mit einem mindestens eine polymerisierbare Doppelbindung enthaltenden Monomer B, das mindestens eine Phosphonatgruppe enthält, durch radikalische Polymerisation der (Meth)Acrylsäureeinheit und der Doppelbindung unter Herstellung einer Beschichtungszusammensetzung,
• lösen der Beschichtungszusammensetzung in einem Lösungsmittel,
• bereitstellen und entfetten eines Implantats,
• aufbringen der Lösung der Beschichtungszusammensetzung auf das Implantat und
• binden der Beschichtungszusammensetzung an das Implantat unter Einwirkung von Temperaturen zwischen 50 und 200 °C.

10. Verfahren nach Anspruch 9, **gekennzeichnet durch** einen Waschschritt und einen anschließenden Trocknungsschritt nach dem Binden der Beschichtungszusammensetzung.

11. Verfahren nach Anspruch 9 oder 10, **dadurch gekennzeichnet, dass** das Aufbringen der Lösung der Beschichtungszusammensetzung durch Spin Coating, ein Tauch- oder Sprühverfahren ausgeführt wird.

12. Verfahren nach einem der Ansprüche 9 bis 11, **dadurch gekennzeichnet, dass** eine Konzentration der Beschichtungszusammensetzung im Lösungsmittel 2 bis 20 mg/ml, insbesondere 8 bis 12 mg/ml, beträgt.

13. Antibakteriell beschichtetes Implantat, hergestellt nach einem Verfahren nach einem der Ansprüche 9 bis 12, wobei das Implantat aus Titan, Zirkon, Tantal, Edelstahl oder Zirkonoxid besteht und eine durchschnittliche Schichtdicke der antibakteriellen Beschichtung 5 bis 50 nm und insbesondere 15 bis 25 nm beträgt.

## Claims

1. A process for producing an antibacterial coating composition for implants comprising the steps of:
• converting a (meth)acrylic acid-based monomer A containing at least one epoxide with a polyguanidine, by the reaction of an amino group of the polyguanidine with the epoxide to obtain a (meth) acrylic acid polyguanidine macromolecule and
• polymerising the (meth)acrylic acid polyguanidine macromolecule with a monomer B containing at least one polymerisable double bond and containing at least one phosphonate group by radical polymerisation of the (meth)acrylic acid unit and the double bond.

2. A process according to Claim 1, **characterised in that** monomer A is 2,3-epoxypropyl methacrylate.

3. A process according to Claim 1 or 2, **characterised in that** monomer B is diethyl(4-vinylbenzyl)phosphonate, 2-(dimethoxyphosphoryl)ethyl methacrylate or 2-(dimethoxyphosphoryl)methyl methacrylate.

4. A process according to one of the preceding claims, **characterised in that** the polyguanidine is poly-2-(2-ethoxy)-ethoxyethyl-guanidium-hydrochloride.

5. A process according to one of the preceding claims, **characterised in that** the polyguanidine is produced by converting guanidium hydrochloride with 1,2-bis(2-aminoethoxy)ethane and having at least one of the following formulas: 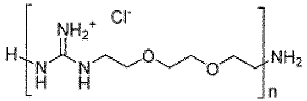 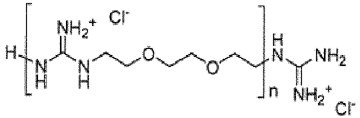 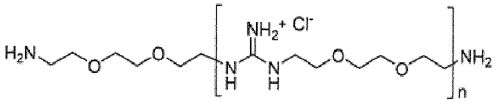 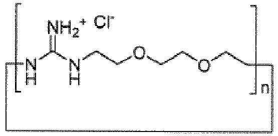 where n is an integer between 1 and 10, preferably between 3 and 6, and in particular 5.

6. A process according to one of Claims 4 or 5, **characterised in that** the molecular weight of the polyguanidine is 800 to 1300 g/mol.

7. A process according to one of the preceding claims, **characterised in that** a proportion of monomer B relating to the antibacterial coating composition is about 67 times the percentage.

8. An antibacterial implant coating composition produced according to a process according to any one of the preceding claims.

9. A process for coating an implant with an antibacterial coating comprising the steps of:
• converting a (meth)acrylic acid-based monomer A containing at least one epoxide with a polyguanidine, by converting an amino group of the polyguanidine with the epoxide producing a (meth)acrylic acid polyguanidine macromolecule,
• polymerising the (meth)acrylic acid polyguanidine macromolecule with a monomer B containing at least one polymerisable double bond containing at least one phosphonate group by radical polymerisation of the (meth)acrylic acid unit and the double bond by producing a coating composition,
• dissolving the coating composition in a solvent,
• providing and degreasing an implant,
• applying the solution of the coating composition to the implant, and
• bonding the coating composition to the implant under the influence of temperatures between 50°C and 200°C.

10. A process according to Claim 9, **characterised by** a washing step and a subsequent drying step after binding of the coating composition.

11. A process according to Claim 9 or 10, **characterised in that** the solution of the coating composition is applied by spin coating, a dipping, or a spraying process.

12. A process according to one of Claims 9 to 11, **characterised in that** a concentration of the coating composition in the solvent is 2 to 20 mg/ml, in particular 8 to 12 mg/ml.

13. An antibacterially coated implant produced by a process according to one of Claims 9 to 12, wherein the implant is made of titanium, zirconium, tantalum, stainless steel, or zirconium oxide and an average coating thickness of the antibacterial coating is 5 to 50 nm, and in particular 15 to 25 nm.

## Revendications

1. Procédé de fabrication d'une composition de revêtement antibactérienne pour implants, comprenant les étapes de :
- conversion d'un monomère A à base d'acide (méth)acrylique, qui contient au moins un époxyde avec une polyguanidine, par réaction d'un groupe amino de la polyguanidine avec l'époxyde, ce qui permet d'obtenir une macromolécule de polyguanidine - acide (méth)acrylique et
- polymérisation de la macromolécule de polyguanidine - acide (méth)acrylique avec un monomère B contenant au moins une double liaison polymérisable, qui contient au moins un groupe phosphonate, par polymérisation radicalaire du motif acide (méth)acrylique et de la double liaison.

2. Procédé selon la revendication 1, **caractérisé en ce que** le monomère A est le 2,3-époxypropylméthacrylate.

3. Procédé selon la revendication 1 ou 2, **caractérisé en ce que** le monomère B est le diéthyl(4-vinylbenzyl)phosphonate, le 2-(diméthoxy-phosphoryl)éthylméthacrylate ou le 2-(diméthoxyphosphoryl)méthylméth-acrylate.

4. Procédé selon l'une des revendications précédentes, **caractérisé en ce que** la polyguanidine est le chlorhydrate de poly-2-(2-éthoxy)-éthoxyéthylguanidine.

5. Procédé selon l'une des revendications précédentes, **caractérisé en ce que** la polyguanidine est fabriquée par conversion du chlorhydrate de guanidine avec du 1,2-bis(2-aminoéthoxy)éthane et présente au moins l'une des formules suivantes : 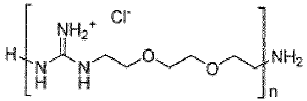 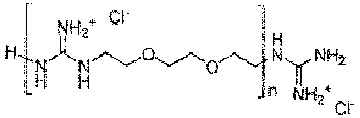 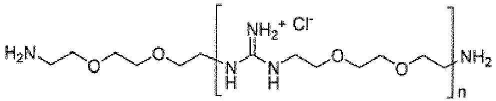 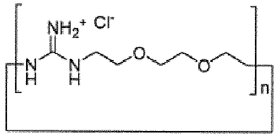 dans laquelle n est un nombre entier compris entre 1 et 10, de préférence entre 3 et 6, et en particulier 5.

6. Procédé selon l'une des revendications 4 ou 5, **caractérisé en ce que** le poids moléculaire de la polyguanidine est de 800 à 1300 g/mole.

7. Procédé selon l'une des revendications précédentes, **caractérisé en ce qu'**une proportion du monomère B par rapport à la composition de revêtement antibactérienne atteint environ 67 % en moles.

8. Composition de revêtement antibactérienne pour implants, fabriquée d'après un procédé selon l'une des revendications précédentes.

9. Procédé de revêtement d'un implant avec un revêtement antibactérien, comprenant les étapes de :
- conversion d'un monomère A à base d'acide (méth)acrylique, qui contient au moins un époxyde avec une polyguanidine, par réaction d'un groupe amino de la polyguanidine avec l'époxyde, ce qui permet d'obtenir une macromolécule de polyguanidine - acide (méth)acrylique,
- polymérisation de la macromolécule de polyguanidine - acide (méth)acrylique avec un monomère B contenant au moins une double liaison polymérisable, qui contient au moins un groupe phosphonate, par polymérisation radicalaire du motif acide (méth)acrylique et de la double liaison, ce qui permet de fabriquer une composition de revêtement,
- solubilisation de la composition de revêtement dans un solvant,
- préparation et dégraissage d'un implant,
- application de la solution de composition de revêtement sur l'implant et
- liaison de la composition de revêtement à l'implant sous l'action de températures comprises entre 50 et 200 °C.

10. Procédé selon la revendication 9, **caractérisé par** une étape de lavage et par une étape de séchage consécutive après la liaison de la composition de revêtement.

11. Procédé selon la revendication 9 ou 10, **caractérisé en ce que** l'application de la solution de composition de revêtement est exécutée par dépôt par centrifugation, un procédé d'immersion ou de pulvérisation.

12. Procédé selon l'une des revendications 9 à 11, **caractérisé en ce qu'**une concentration de la composition de revêtement dans le solvant est de 2 à 20 mg/ml, en particulier de 8 à 12 mg/ml.

13. Implant enduit d'un revêtement antibactérien, fabriqué d'après un procédé selon l'une des revendications 9 à 12, dans lequel l'implant est constitué de titane, de zircone, de tantale, d'acier ou d'oxyde de zircone et une épaisseur de couche moyenne du revêtement antibactérien est de 5 à 50 nm, et en particulier de 15 à 25 nm.
